Europäisches Patentamt

European Patent Office

Office européen des brevets

(19)

(11) Veröffentlichungsnummer: **0 022 509**
**B2**

(12) **NEUE EUROPÄISCHE PATENTSCHRIFT**

(45) Veröffentlichungstag der neuen Patentschrift:
09.04.86

(21) Anmeldenummer: 80103717.7

(22) Anmeldetag: 01.07.80

(51) Int. Cl.⁴: **C 07 C 43/04**, C 07 C 11/09, C 07 C 41/06, C 07 C 7/148

(54) Verfahren zur gemeinsamen Herstellung von Methyl-tert.-butyläther und Gewinnung von Isobuten.

(30) Priorität: 14.07.79 DE 2928509

(43) Veröffentlichungstag der Anmeldung:
21.01.81 Patentblatt 81/3

(45) Bekanntmachung des Hinweises auf die Patenterteilung:
19.01.83 Patentblatt 83/3

(45) Bekanntmachung des Hinweises auf die Entscheidung über den Einspruch:
09.04.86 Patentblatt 86/15

(84) Benannte Vertragsstaaten:
AT BE CH DE FR GB IT LI NL SE

(73) Patentinhaber: BASF Aktiengesellschaft,
Carl-Bosch-Strasse 38, D-6700 Ludwigshafen (DE)

(72) Erfinder: Volkamer, Klaus, Dr., Heidelberger Ring 21,
D-6710 Frankenthal (DE)
Erfinder: Lindner, Alfred, Dr., Ringstrasse 22,
D-6712 Bobenheim-Roxheim 1 (DE)
Erfinder: Merger, Franz, Dr., Max-Slevogt-Strasse 25,
D-6710 Frankenthal (DE)
Erfinder: Wagner, Ulrich, Dr., Knospstrasse 7,
D-6703 Limburgerhof (DE)
Erfinder: Brunner, Erwin, Dr., Neuhausener Weg 1,
D-6700 Ludwigshafen (DE)
Erfinder: Sandrock, Gerhard, Dr.,
Albrecht-Duerer-Ring 36C, D-6710 Frankenthal (DE)
Erfinder: Strohmeyer, Max, Dr., Woogstrasse 41,
D-6703 Limburgerhof (DE)

(56) Entgegenhaltungen:
EP - A - 0 015 513
AT - B - 338 223
AT - B - 346 297
DE - A - 2 705 538
DE - A - 2 706 465
DE - A - 2 802 198
DE - B - 1 216 865
DE - B - 1 934 422
DE - B - 2 011 826
US - A - 4 148 695

Hydrocarbons from Petroleum, 1953, p. 86/87
Azeotropic Data-III, Advances in Chemistry, Series 116, 1973 Tab. 1 p. 81
Journal of Catalysis 46, p. 49-57 (1977)

**Beschreibung**

Die Erfindung betrifft ein Verfahren zur gemeinsamen Herstellung von Methyl-tert.-butyläther und Gewinnung von Isobuten aus Isobuten enthaltenden $C_4$-Kohlenwasserstoffgemischen.

Es ist bereits bekannt, z.B. aus der DE—OS 2 629 769, Methyl-tert.-butyläther durch Umsetzung eines Isobuten enthaltenden $C_4$-Kohlenwasserstoffgemisches mit Methanol herzustellen, Bei der Herstellung des Methyl-tert.-butyläthers, das in zunehmendem Maße als Kraftstoffadditiv zur Oktanzahlverbesserung Verwendung findet, wird in Kauf genommen, daß bei der destillativen Abtrennung der nicht umgesetzten Kohlenwasserstoffe aus dem Reaktionsgemisch die abgetrennten Kohlenwasserstoffe aufgrund von Kohlenwasserstoff/Methanol-Azeotropen etwa 2% Methanol enthalten, das nur durch aufwendige Verfahren, z.B. durch Einschaltung einer Wasserwäsche, zurückgewonnen werden kann.

Es ist weiter, z.B. aus der DE—PS 1 216 865, DE—AS 1 934 422 oder DE—AS 2 011 826, ein Verfahren zur Gewinnung von Isobuten bekannt, bei dem in einer ersten Stufe ein Isobuten enthaltendes $C_4$-Kohlenwasserstoffgemisch mit Methanol umgesetzt wird und der gebildete Methyl-tert.-butyläther in einer zweiten Stufe in Methanol und Isobuten zerlegt wird. Die bekannten Verfahren zur Isobutengewinnung haben jedoch den Nachteil, daß zusätzlich zu der bereits vorstehend beschriebenen Bildung von azeotropen Gemischen aus Methanol und den nicht umgesetzten Kohlenwasserstoffen in der Stufe der Ätherbildung auch bei der destillativen Auftrennung des aus Isobuten und Methanol bestehenden Reaktionsgemisches der Zerlegungsstufe Methanol und Isobuten ein azeotropes Gemisch bilden, so daß auch in der Zerlegungsstufe die Abtrennung des Methanols sehr erschwert ist und beispielsweise eine aufwendige Wasserwäsche eingeschaltet werden muß. Die bekannten Verfahren zur Isobutengewinnung unter Zerlegung des in einer ersten Verätherungsstufe erhaltenen tertiären Äthers haben wegen der genannten Nachteile keine technische Anwendung gefunden.

Darüber hinaus werden in den europäischen Patentanmeldungen EP—$A_2$—0 003 305 und EP—$A_1$—0 015 513 Verfahren zur Gewinnung von Isobuten aus Isobuten enthaltenden $C_4$-Kohlenwasserstoffgemischen vorgeschlagen, bei denen zunächst in einer Verätherungsstufe das $C_4$-Kohlenwasserstoffgemische in Gegenwart eines sauren Kondensationsmittels mit einem primären $C_3$- oder $C_4$-Alkohol umgesetzt wird und anschließend der erhaltene tertiäre Äther in Gegenwart eines sauren Katalysators bei erhöhten Temperaturen in Isobuten und primären Alkohol zerlegt wird.

In technischem Maßstab wird Isobuten aus $C_4$-Kohlenwasserstoffgemischen bisher nur durch Anwendung von Schwefelsäure-Extraktionsverfahren gewonnen. Bei diesen Schwefelsäure-Extraktionsverfahren muß jedoch Schwefelsäure hoher Konzentration verwendet werden, so daß die Verwendung kostspieliger Materialien für die Ausrüstung erforderlich ist. Da weiterhin Nebenreaktionen des Isobutens, beispielsweise Dimerisation, Polymerisation, Hydratation und dergl. während der Extraktion erfolgen, ist das Schwefelsäure-Extraktionsverfahren hinsichtlich Ausbeute und Qualität der Produkte nicht stets zufriedenstellend.

Der Erfindung liegt die Aufgabe zugrunde, ein Verfahren zur gemeinsamen Herstellung von Methyl-tert.-butyläther und Gewinnung von Isobuten aus Isobuten enthaltenden $C_4$-Kohlenwasserstoffen zur Verfügung zu stellen.

Es wurde nun ein einfaches Verfahren gefunden, zur gemeinsamen Herstellung von Methyl-tert.-butyläther und Gewinnung von Isobuten aus Isobuten enthaltenden $C_4$-Kohlenwasserstoffgemischen, welches dadurch gekennzeichnet ist, daß man

a) das $C_4$-Kohlenwasserstoffgemisch in Gegenwart eines sauren Kondensationsmittels in einer oder mehreren Verätherungsstufen mit Methanol und einem primären $C_3$- oder $C_4$-Alkohol umsetzt,

b) das erhaltene Reaktionsgemisch bzw. die erhaltenen Reaktionsgemische anschließend durch Destillation in

— eine die nicht umgesetzen Kohlenwasserstoffe enthaltende Fraktion,

— den Methyl-tert.-butyläther und

— eine den $C_3$- oder $C_4$-Alkyl-tert.-butyläther enthaltende Fraktion auftrennt und

c) dann den $C_3$- oder $C_4$-Alkyl-tert.-butyläther in Gegenwart eines sauren Katalysators bei erhöhten Temperaturen in Isobuten und primären $C_3$- oder $C_4$-Alkohol zerlegt.

Nach dem neuen Verfahren läßt sich aus dem nach der Verätherungsstufe erhaltenen Reaktionsgemische ein praktische $C_3$- oder $C_4$-Alkoholfreies $C_4$-Kohlenwasserstoff-Raffinat durch einfache Destillation ohne Einschaltung einer Wasserwäsche abtrennen, da nicht umgesetzter primärer $C_3$- oder $C_4$-Alkohol überraschenderweise keine Azeotrope mit den $C_4$-Kohlenwasserstoffen bildet. Im allgemeinen beträgt die Konzentration an $C_3$- oder $C_4$-Alkohol im $C_4$-Kohlenwasserstoff-Raffinat höchstens 1000 Gew.-ppm, vorzugsweise höchstens 200 Gew.-ppm, insbesondere höchstens 20 Gew.-ppm. Es war weiter überraschend, daß das bei dem Verfahren nach der Erfindung durch Destillation ohne Einschaltung einer Wasserwäsche erhaltene $C_4$-Kohlenwasserstoff-Raffinat eine geringere Methanol-Konzentration aufweist als wie sie bei der Verätherung mit Methanol allein aufgrund der vorstehend beschriebenen Bildung von Kohlenwasserstoff/Methanol-Azeotropen erhalten wird. Im allgemeinen wird nach dem Verfahren gemäß der Erfindung ein $C_4$-Kohlenwasserstoff-Raffinat mit einem Methanolgehalt von weniger als 1 Gew.-%, vorzugsweise weniger als 0,5 Gew.-% abgezogen. Auch bei der destillativen Trennung des bei der Zerlegung des $C_3$- oder $C_4$-Alkyl.-tert.-butyläthers erhaltenen Reaktionsgemisches in Isobuten und den $C_3$- oder $C_4$-Alkohol werden

überraschenderweise keine Azeotrope des Alkohols mit Isobuten gebildet. Das Isobuten kann deshalb in großer Reinheit erhalten werden. Im allgemeinen wird das Isobuten als Kopfprodukt der Destillation mit einem Gehalt an $C_3$- oder $C_4$-Alkohol von höchstens 100 Gew.-ppm, vorzugsweise höchstens 50 Gew.-ppm, insbesondere höchstens 20 Gew.-ppm, abgezogen. Der $C_3$- oder $C_4$-Alkohol kann daher in einfacher Weise ohne Einschaltung einer Wasserwäsche praktisch ohne Verluste zurückgewonnen und in die Verätherungsstufe zurückgeführt werden.

Ein weiterer Vorteil des vorliegenden Verfahrens liegt in der großen Flexibilität der Anlage zur Nutzung des im $C_4$-Ausgangskohlenwasserstoffgemische enthaltenen Isobutens, die leicht Anpassungen an weschselnde Nachfragen des Marktes bezüglich Isobuten und Methyl-tert.-butyläther erlaubt.

Das Isobuten wird nach dem Verfahren der Erfindung in hoher Ausbeute, bezogen auf den eingesetzten primären $C_3$- oder $C_4$-Alkohol, erhalten. Es war überraschend, daß diese hohe Ausbeute mit einem höheren Alkohol wie einem primeren $C_3$- oder $C_4$-Alkohol möglich war, da aus der US—PS 3 170 000, insbesondere Tabelle 1 bekannt ist, daß bei der Umsetzung von $C_5$-Kohlenwasserstoffgemischen mit Alkoholen bei Verwendung von $C_3$- oder $C_4$-Alkoholen wesentlich schlechtere Ausbeuten an tertiärem Äther erhalten werden als bei der Verwendung von Äthanol oder Methanol.

Für das erfindungsgemäße Verfahren geeignete Isobuten enthaltende $C_4$-Kohlenwasserstoffgemische werden beispielsweise beim thermischen oder katalytischen Cracken von Erdölprodukten, bei der Herstellung von Äthylen durch Pyrolyse von verflüssigtem Petroleumgas (LPG), Leichtbenzin (Naphtha), Gasöl oder dergl. oder bei der katalytischen Dehydrierung von n-Butan und/oder n-Buten erhalten. Diese $C_4$-Kohlenwasserstoffgemische enthalten in der Regel neben dem isobuten olefinische und paraffinische $C_4$-Kohlenwasserstoff und können darüber hinaus noch Butadien, z.B. in einer Menge von bis zu 70 Gew.-%, und höhere Acetylene, wie Butin-1 und Butenin enthalten. Butadien enthaltende $C_4$-Kohlenwasserstoffgemische können als solche oder nach vorheriger Abtrennung des Butadiens aus dem $C_4$-Kohlenwasserstoffgemische, z.B. durch Butadien-Extraktion unter Verwendung eines selektiven Lösungsmittels verwendet werden. Die $C_4$-Kohlenwasserstoffgemische können außerdem noch $C_3$-Kohlenwasserstoffe wie Propan, Propen, Propin, z.B. bie zu 10 Gew.-%, enthalten. Die $C_4$-Kohlenwasserstoffgemische weisen im allgemeinen einen Isobutengehalt von 5 bis 95 Gew.-%, vorzugsweise 10 bis 90 Gew.-%, insbesondere 20 bis 70 Gewichtsprozent, auf. Vorzugsweise werden solche $C_4$-Kohlenwasserstoffgemische verwendet, die neben Isobuten n-Butan, Isobutan, Buten-1, trans-Buten-2 und cis-Buten-2 und ggf. Butadien-1,3 enthalten.

Als erfindungsgemäß zu verwendende primäre $C_3$- oder $C_4$-Alkohole (d.h. Alkohole mit 3 oder 4 Kohlenstoffatomen) werden im allgemeinen n-Propanol, n-Butanol oder Isobutanol, vorzugsweise n-Propanol oder Isobutanol, und insbesondere Isobutanol eingesetzt. Die $C_3$- oder $C_4$-Alkohole und das erfindungsgemäß einzusetzende Methanol werden z.B. als technische Produkte überlicher Reinheit, beispielsweise mit einer Reinheit von mindestens 95%, vorzugsweise mindestens 98% verwendet.

Als saure Kondensationsmittel für die Verätherung, die oie erste Stufe darstellt, kommen beispielsweise Mineralsäuren wie Schwefelsäure, Phosphorsäure, organische Sulfonsäuren wie Benzolsulfonsäure, p-Toluolsulfonsäure, saure Aluminiumsalze oder saure Katalysatoren vom Friedel-Crafts-Typ wie Kupfer(II)-chlorid, Eisen(III)-chlorid sowie vorzugsweise Ionenaustauscher in der Wasserstofform in Betracht. Geeignete Ionenaustauscher sind beispielsweise sulfonierte Kohlen, sulfonierte Phenol-Formaldehyd-Harze, sulfonierte von Cumaron-Inden-Kondensationsprodukten abgeleitete Harze sowie insbesondere sulfonierte Polystyrol-Harze wie Kern-sulfonierte vernetzte Styrol-Divinylbenzol-Copolymerisate. Bei Verwendung eines flüssigen oder gelösten sauren Kondensationsmittels beträgt die Menge des flüssigen oder gelösten sauren Kondensationsmittels im allgemeinen etwa 0,001 bis 0,9 l, vorzugsweise 0,01 bis 0,71 pro l Reaktorvolumen. Bei Verwendung von festen sauren Kondensationsmitteln beträgt die Menge des festen Kondensationsmittels im allgemeinen 0,01 bis 1 l Schüttvolumen pro l Reaktorvolumen. Die festen sauren kondensationsmittel können als solche oder auf einem Trägermaterial verwendet werden. Geeignete Trägermaterialien sind z.B. Aluminiumoxid, Kieselsäure, Aktivkohle, organische Polymere. Für die Verätherung können z.B. Rührkessel- oder Festbettreaktoren verwendet werden. Vorzugsweise werden Festbettreaktoren eingesetzt.

Für die Verätherung wird das $C_4$-Kohlenwasserstoffgemisch mit dem primären $C_3$- oder $C_4$-Alkohol und Methanol in Gegenwart des sauren Kondensationsmittels im allgemeinen bei Temperaturen von 20 bis 120°C, vorzugsweise 20 bis 100°C, insbesondere 30 bis 80°C umgesetzt. Mit besonderem Vorteil wird der Temperaturverlauf in der Verätherungsstufe so gewählt, daß die Austrittstemperatur des Reaktionsgemisches aus der Verätherungsstufe 20 bis 100°C, vorzugsweise 20 bis 65°C, insbesondere 30 bis 50°C beträgt. Vorzugsweise liegt die Austrittstemperatur niedriger als die Reaktionstemperatur im mittleren und/oder Anfangsbereich der Verätherungsstufe.

Die erfindungsgemäße Verätherung kann bei Normaldruck erfolgen. Es ist jedoch zweckmäßig, einen geringen Überdruck, z.B. Drücke von 1,01 bis 30 bar, insbesondere 2 bis 20 bar, anzuwenden. Das Isobuten enthaltende $C_4$-Kohlenwasserstoffgemische kann dabei je nach Druck und Temperatur für die Umsetzung flüssig oder gasförmig eingesetzt werden. Vorzugsweise werden flüssige Isobuten enthaltende $C_4$-Kohlenwasserstoffgemische engesetzt. Die Verätherung kann diskontinuierlich durchgeführt werden. Die Reaktionszeiten liegen bei diskontinuierlicher Arbeitsweise im allgemeinen zwischen 1 Minute und 5 Stunden. Vorzugsweise wird die Verätherung jedoch kontinuierlich durchgeführt, wobei das Verhältnis aus Reaktorvolumen in l und dem Durchsatz in l/h im allgemeinen 0,01 bis 5 Stunden, vorzugsweise 0,03 bis 1 Stunde beträgt.

Für die Verätherung werden Methanol und der $C_3$-oder $C_4$-Alkohol im allgemeinen im Gewichtsverhältnis 1:50 bis 50:1, vorzugsweise 1:20 bis 20:1 insbesondere 1:10 bis 10:1 eingesetzt.

Das Molverhältnis von Methanol und primärem $C_3$- oder $C_4$-Alkohol zu dem im $C_4$-Kohlenwasserstoffgemische enthaltenen Isobuten beträgt bei der Verätherung im allgemeinen 100:1 bis 0,7:1, vorzugsweise 20:1 bis 0,9:1,4:1 bis 1:1.

Die Verätherung kann in einer oder mehreren hintereinandergeschalteten Reaktionsstufen durchgeführt werden. Bei der Anwendung mehrerer Verätherungsstufen werden im allgemeinen 2 oder 3 Stufen, vorzugsweise 2 Stufen, angewendet.

Das durch Destillation erhaltene $C_4$-Kohlenwasserstoffgemisch-Raffinat aus den nicht umgesetzten $C_4$-Kohlenwasserstoffen enthält ggf. im Überschuß zur Verätherung zugesetztes Methanol, das aufgrund der Bildung von Kohlenwasserstoff/Methanol-Azeotropen bei der destillativen Abtrennung des $C_4$-Kohlenwasserstoffgemische-Raffinats ebenfalls überdestilliert wird. Dagegen ist das Raffinat überraschenderweise praktisch frei von dem ggf. im Überschuß zur Verätherung zugesetzten primären $C_3$- oder $C_4$-Alkohol.

Falls die Verätherung in einer Stufe durchgeführt wird, wird das $C_4$-Kohlenwasserstoffgemische zweckmäßig mit einer Mischung aus Methanol und dem primären $C_3$- oder $C_4$-Alkohol umgesetzt. Falls die Verätherung entsprechend einer bevorzugten Ausführungsform in zwei hintereinandergeschalteten Stufen durchgeführt wird, können in diesen Stufen unterschiedliche Alkohole eingesetzt werden, wobei zorzugsweise in der ersten Verätherungsstufe methanol und in der zweiten Verätherungsstufe der primäre $C_3$- oder $C_4$-Alkohol verwendet werden. Es können jedoch auch in beiden Stufen jeweils Gemische aus Methanol und dem primären $C_3$- oder $C_4$-Alkohol eingesetzt werden. Es ist jedoch auch möglich, in der ersten Stufe das Gemisch der Alkohole und in der zweiten Stufe den primären $C_3$- oder $C_4$-Alkohol bzw. in der ersten Stufe den $C_3$- oder $C_4$-Alkohol oder vorzugsweise Methanol und in der zweiten Stufe das Gemisch der Alkohole einzusetzen.

Vorzugsweise wird bei zweistufiger Arbeitsweise in der ersten Verätherungsstufe das Gemische der Alkohole, z.B. im Molverhältnis 1:20 bis 20:1, vorzugsweise 1:10 bis 10:1, insbesondere 1:5 bis 5:1, eingesetzt, während in der zweiten Verätherungsstufe weitgehend, d.h. mit einem Anteil der $C_3$- oder $C_4$-Alkoholmenge an der gesamten zur zweiten Stufe zugegebenen Alkoholmenge von mindestens 50 Mol-% vorzugsweise mindestens 80 Mol-%, insbesondere mindestens 90 Mol-%, oder ausschließlich primärer $C_3$- oder $C_4$-Alkohol zugesetzt wird.

Aus dem bei der zweistufigen Arbeitsweise und Einsatz von Methanol in der ersten Stufe nach Destillation des Reaktionsgemische der ersten Stufe erhaltenen, die nicht umgesetzten Kohlenwasserstoffe enthaltenden Kopfprodukt kann das im Kopfprodukt enthaltene Methanol vor Überführung des Kopfproduktes in die zweite Verätherungsstufe, z.B. durch eine Wasserwäsche gegebenenfalls mit anschließender Trocknung, entfernt werden. Vorzugsweise wird das Kopfprodukt jedoch ohne Methanolabtrennung in der zweiten Verätherungsstufe weiter umgesetzt.

Das isobuten enthaltende Ausgangs-$C_4$-Kohlenwasserstoffgemische kann bei zweistufiger Arbeitsweise ganz der ersten Stufe zugeführt werden, um dann nach Abtrennung aus dem erhaltenen Reaktionsgemisch der zweiten Verätherungsstufe zugeführt zu werden. Es kann jedoch vorteilhaft sein, das Ausgangs-$C_4$-Kohlenwasserstoffgemisch nur teilweise, z.B. zu 5 bis 95%, der ersten Verätherungsstufe zuzuführen und den verbleibenden Rest des Ausgangs-$C_4$-Kohlenwasserstoffgemischs zweckmäßig nach Vermischen mit den nicht umgesetzen $C_4$-Kohlenwasserstoffen der ersten Stufe der zweiten Verätherungsstufe zuzuführen. Das Reaktionsgemisch der zweiten Verätherungsstufe wird einer Destillation unterworfen, bei der als Kopfprodukt ein die nicht umgesetzen Kohlenwasserstoff enthaltendes $C_4$-Kohlenwasserstoff-Raffinat mit einem Isobuten-Gehalt von im allgemeinen höchstens 5 Gew.-%, vorzugsweise höchstens 3 Gew.-%, insbesondere höchstens 1 Gew.-% abgezogen wird. Es ist jedoch auch möglich, ein $C_4$-Kohlenwasserstoff-Raffinat mit weniger als 0,5 Gew.-% Isobuten abzuziehen.

Bei weitgehender, d.h. bei einem Anteil der $C_3$- oder $C_4$-Alkoholmenge an der gesamten zur zweiten Stufe zugegebenen Alkoholmenge von mindestens 50 Mol-%, vorzugsweise mindestens 80 Mol-%, insbesondere mindestens 90 Mol-%, oder ausschließlicher Zugabe von $C_3$- oder $C_4$-Alkohol zur zweiten Verätherungsstufe, z.B. bei Umsetzung mit dem aus der ersten Verätherungsstufe durch Destillation erhaltenen methanolhaltigen $C_4$-Kohlenwasserstoff-Raffinat, kann die Methanol-Konzentration im durch Destillation des Reaktionsgemische der zweiten Verätherungsstufe erhaltenen $C_4$-Kohlenwasserstoff-Raffinat weit unter die Konzentration gesenkt werden, die der azeotropen Zusammensetzung des Methanols mit den $C_4$-Kohlenwasserstoffen entspricht, ohne daß die Einschaltung einer Wasserwäsche erforderlich ist, Vorzugsweise wird ein $C_4$-Kohlenwasserstoff-Raffinat mit einem Methanolgehalt von weniger als 1 Gew.-%, vorzugsweise weniger als 0,5 Gew-% und einem Gehalt an $C_3$- oder $C_4$-Alkohol, Methyl-tert.-butyläther und $C_3$- oder $_4$-Alkyl-tert.-butyläther von jeweils höchstens 1000 Gew.-ppm, vorzugsweise höchstens 500 Gew.-ppm, insbesondere höchstens 100 Gew.-ppm abgezogen. Soll das $C_4$-Kohlen-

wasserstoff-Raffinat praktisch methanolfrei sein, so kann eine Nachbehandlung des Raffinats, z.B. durch eine Wasserwäsche oder eine Druckdestillation bei einem Druck von im allgemeinen mindestens 10 bar, vorzugsweise 20 bar bis zum kritischen Druck der $C_4$-Kohlenwasserstoffe, nachgeschaltet werden. In der Regel ist jedoch eine solche Nachbehandlung nicht erforderlich.

In einer bevorzugten Ausführungsform des erfindungsgemäßen Verfahrens wird die Verätherungsreaktion in ein oder mehreren, vorzugsweise 2, Verätherungsstufen durchgeführt und bei mehrstufiger Arbeitsweise bereits in der ersten Verätherungsstufe mit Methanol umgesetzt, wobei das Reaktionsgemische der Verätherungsstufe bzw. bei mehrstufiger Arbeitsweise das Reaktionsgemische der ersten Verätherungsstufe einer Destillationszone mit Seitenabzug zugeführt werden, wobei aus dem Seitenabzug eine Methanol-reiche Fraktion abgezogen wird, welche zweckmäßig in die Verätherungsstufe bzw. bei mehrstufiger Arbeitsweise in die erste Verätherungsstufe zurückgeführt wird. Es ist jedoch auch möglich, die Destillation ohne Seitenabzug durchzuführen.

Bei zweistufiger Arbeitsweise wird als Kopfprodukt der ersten Destillation zweckmäßig eine die nicht umgesetzten Kohlenwasserstoff enthaltende Fraktion abgezogen und der zweiten Verätherungsstufe zugeführt, die im allgemeinen 1 bis 5000 Gew.-ppm, vorzugsweise 10 bis 2000 Gew.-ppm, insbesondere 50 bis 1000 Gew.-ppm Methyl-tert.-butyläther enthält. Außerdem enthält das Kopfprodukt bei üblicher Destillation in der Regel mehr als 1,5% Methanol. Das Methanol kann z.B. durch eine Wasserwäsche entfernt werden. Im allgemeinen wird das Raffinat der ersten Stufe jedoch direkt, also ohne Einschaltung einer Wasserwäsche der zweiten Stufe zugeführt.

Das nach der destillativen Abrennung des $C_4$-Kohlenwasserstoffgemische-Raffinats verbleibende Bodenprodukt, das noch Methyl-tert.-butyläther, ggf. Methanol, primären $C_3$- oder $C_4$-Alkohol und den $C_3$- oder $C_4$-Alkyl-tert.-butyläther enthält, wird einer weiteren Destillation unterworfen, wobei der Methyl-tert.-butyläther und ggf. vorhandenes Methanol abgetrennt und schließlich ein den $C_3$- oder $C_4$-Alkyl-tert.-butyläther und ggf. $C_3$- oder $C_4$-Alkokol enthaltendes Bodenprodukt erhalten wird. Im allgemeinen enthält das erhaltene Bodenprodukt jeweils weniger als 500 Gew.-ppm, vorzugsweise weniger als 100 Gew.-ppm, insbesondere weniger als 10 Gew.-ppm Methanol und Methyl-tert.-butyläther.

Der erhaltene $C_3$- oder $C_4$-Alkyl-tert.-butyläther wird anschließend in der zweiten Stufe des Verfahrens in Gegenwart eines sauren Katalysators bei erhöhten Temperaturen in Isobuten und primären $C_3$- oder $C_4$-Alkohol zerlegt. Als Ausgangsprodukt für die Zerlegung kann tertiärer Äther, der praktisch frei von $C_3$- oder $C_4$-Alkohol ist, verwendet werden, der beispielsweise durch Verwendung einer Menge an $C_3$- oder $C_4$-Alkohol bei der Verätherung, die höchstens der stöchiometrische erforderlichen Alkoholmenge entspricht, und durch Abtrennung, z.B. durch Abdestillieren, von überschüssig zugesetztem primärem $C_3$- oder $C_4$-Alkohol aus dem nach der Destillation des Reaktionsgemischs der Verätherung schließlich erhaltenen Bodenprodukt erhalten worden ist. Vorzugsweise wird der nach der destillativen Abrennung des $C_4$-Kohlenwasserstoffgemisch-Raffinats und des Methyl-tert.-butyläthers als Bodenprodukt erhaltene $C_3$- oder $C_4$-Alkyl-tert.-butyläther ohne weitere Abtrennung von ggf. vorhandenem überschüssigem $C_3$- oder $C_4$-Alkohol für die Zerlegung eingesetzt. Es is jedoch auch möglich, nur einen Teil des überschüssigen $C_3$- oder $C_4$-Alkohols abzutrennen. Zweckmäßig enthält der als Bodenprodukt erhaltene $C_3$- oder $C_4$-Alkyl-tert.-butyläther weniger als 1000 Gew.-ppm, vorzugsweise weniger als 100 Gew.-ppm, insbesondere weniger als 10 Gew.-ppm an den nicht umgesetzten $C_4$-Kohlenwasserstoffen.

Für die Zerlegung wird der tertiäre Äther verdampft und mit dem sauren Katalysator in der Dampfphase Kontaktiert. Als saure Katalysatoren kommen beispielsweise Ionenaustauscher in der Wasserstofform, wie sulfonierte Kohlen, sulfonierte Phenol-Formaldehydharze, sulfonierte von Cumaron-Inden-Kondensationsprodukten abgeleitete Harze sowie insbesondere sulfonierte Polystyrolharze wie Kern-sulfonierte, vernetzte Styrol-Divinylbenzol-Copolymerisate in Betracht.

Weiter sind feste Phosphorkatalysatoren, die Mono- oder vorzugsweise Polyphosphorsäure auf einem festen Trägermaterial enthalten, vorteilhaft geeignet. Geeignete Trägermaterialien für die Phosphorsäurekatalysatoren sind z.B. Aluminiumoxid, Kieselsäure, Aktivkohle, Kieselgur oder Bims. Vorzugsweise wird Keiselgel als Trägermaterial verwendet.

Weitere geeignete Katalysatoren sind saure Metallsulfate wie Natriumhydrogensulfat, Calciumhydrogensulfat, Aluminiumsulfate, Nickelsulfat, Kupfersulfat, Kupfersulfat, Kobaltsulfat, Cadmiumsulfat, Strontiumsulfat. Diese sauren Metallsulfate können als solche verwendet werden. Vorzugsweise werden sie auf einem Trägermaterial angewendet. Geeignete Trägermaterialien sind z.B. Kieselgel, Aktivkohle, Aluminiumoxid oder Bims.

Weiter kommen Kieselgel oder Aluminiumoxid alleine als Katalysatoren für die Zerlegung in Betracht.

In einer weiteren Ausführungsform des erfindungsgemäßen Verfahrens wird für die Zerlegung als saurer Katalysator ein Metallphosphat, insbesondere ein Metallhydrogenphosphat, verwendet. Diese Phosphate können Phosphorsäure auch im Überschuß, der über die stöchiometrische Zusammensetzung der sauren Metallphosphate hinausgeht, enthalten, z.B. in einem Überschuß bis zu 65%, vorzugsweise bis zu 20%, insbesondere bis zu 10%. Als derartige Metallphosphate können beispeilsweise Magnesiumphosphate, Calciumphosphate, Strontiumphosphate, Bariumphosphate, Manganphosphate, Nickelphosphate, Kupferphosphate, Kobaltphosphate, Cadmiumphosphate, Eisen(II)-phosphate, Chromphosphate und insbesondere Aluminiumphosphate verwendet werden. Der Metallphosphat-Katalysator kann als solcher oder auf einem Trägermaterial verwendet werden. Geeignete Trägermaterialien sind beispeilsweise Aluminiumoxid, Kieselsäure, Aktivkohle, Zinkoxid.

Die Menge des sauren Katalysators beträgt im allgemeinen etwa 0,01 bis 1 kg, vorzugsweise etwa 0,03 bis 0,3 kg je 1 kg/h Durchsatz des teritären Äthers durch den Reaktor. Vorzugsweise werden für die Zerlegung des tertiären Äthers Festbettreaktoren verwendet.

Die Zerlegungstemperatur des tertiären Äthers variiert in Abhängigkeit von der Art des sauren Katalysators und der Kontaktzeit, liegt jedoch im allgemeinen bei Temperaturen von 50 bis 350°C, vorzugsweise 80 bis 300°C, insbesondere 100 bis 250°C. Bei der Verwendung von Metallphosphaten oder Phosphorkatalysatoren als Zerlegungskatalysatoren werden im allgemeinen Temperaturen von 80 bis 350°C, vorzugsweise 90 bis 260°C, insbesondere 100 bis 250°C angewendet.

Die Kontaktzeit des verdampften tertiären Äthers beträgt zweckmäßig, 0,1 bis 20 Sekunden, vorzugsweise 1 bis 10 Sekunden.

Die Zerlegung des tertiären Äthers kann bei Normaldruck erfolgen. Es is jedoch auch möglich, bzw. es kann auch vorteilhaft sein, Überdruck anzuwenden, z.B. Drücke von 2 bis 15 bar, vorzugsweise 3 bis 12 bar, insbesondere Drücke von 4 bis 12 bar. Die Zerlegung kann jedoch auch bei vermindertem Druck durchgeführt werden. Die Zerlegung des tertiären Äthers kann diskontinuierlich erfolgen. Vorzugsweise wird sie jedoch kontinuierlich durchgeführt.

Das bei der Zerlegung erhaltene Reaktionsgemisch, welches als Reaktionsproduckt Isobuten und primären $C_3$- oder $C_4$-Alkohol enthält, wird in Isobuten und den $C_3$- oder $C_4$-Alkohol aufgetrennt. Zweckmäßig erfolgt diese Auftrennung durch Destillation, wobei in einfacher Weise, ohne Einschaltung einer Wasserwäsche, ein hochreines Isobuten mit einer Reinheit von mehr als 99,3%, vorzugsweise von mehr als 99,8% erhalten werden kann. Zweckmäßig weist das ohne Einschaltung einer Wasserwäsche erhaltene hochreine Isobuten einen Gehalt an dem $C_3$- oder $C_4$-Alkohol von höchstens 500 Gew.-ppm, vorzugsweise höchstens 200 Gew.-ppm, insbesondere höchstens 20 Gew.-ppm auf, wobei ohne besonderen destillativen Trennaufwand auch Gehalte an $C_3$- oder $C_4$-Alkohol von höchstens 5 Gew.-ppm erhalten werden können.

Der aus der Aftrennung erhaltene $C_3$- oder $C_4$-Alkohol wird im allgemeinen der Verätherung wieder zugeführt. Der in die Verätherung zurückgeführt $C_3$- oder $C_4$-Alkohol kann durch Anreicherung ohne besondere Nachteile für die Verätherung bis zu 30 Gew.-%, vorzugsweise bis zu 15 Gew.-%, insbesondere bis zu 10 Gew.-% Di-$C_3$- oder $C_4$-Alkyläther sowie gegebenenfalls bis zu je 10 Gew.-%, vorzugsweise bis zu je 5 Gew.-%, insbesondere bis zu je 2 Gew.-% $C_3$- oder $C_4$-

Alkyl-tert.-butyläther und/oder Isobuten enthalten.

Bei kontinuierlicher Arbeitsweise und Rückführung des nach der Zerlegung des tertiären Äthers und anschließender Aufarbeitung des Reaktionsgemisches erhaltenen primären $C_3$- oder $C_4$-Alkohols in die Verätherungszone kann es bei dem neuen Verfahren zweckmäßig sein, bei der Verwendung von Isobutanol als $C_4$-Alkohol aus dem Isobutanolstrom zur Entfernung von eventuell angereicherten Verunreinigungen, wie Diisobutyläther, einen Isobutanolteilstrom abzuzweigen. Zweckmäßig wird aus dem Isobutanolstrom ein Teilstrom abgezweigt, der 0,1 bis 10 Gew.-%, vorzugsweise 0,5 bis 5 Gew.-% des Isobutanolstroms beträgt und im allgemeinen 3 bis 40 Gew.-%, vorzugsweise 5 bis 35 Gew.-%, insbesondere 10 bis 30 Gew.-%, Diisobutyläther enthält. In einer vorteilhaften Ausführungsform des Verfahrens wird der Isobutanolteilstrom in an sich bekannter Weise in Gegenwart eines Dehydatisierungskatalysators zu Isobuten dehydratisiert, wodurch die Ausbeute an Isobuten anders als bei den bekannten Verfahren zusätzlich erhöht wird.

Zweckmäßig wird die Dehydratisierung in der Gasphase an einem Katalysator durchgeführt. Geeignete Katalysatoren sind z.B. Kieselgel, Thoriumoxid, Titan(IV)-oxid und insbesondere Aluminiumoxid. Im allgemeinen werden für die Dehydratisierung Temperaturen von 250 bis 450°C, vorzugsweise 300 bis 400°C angewendet.

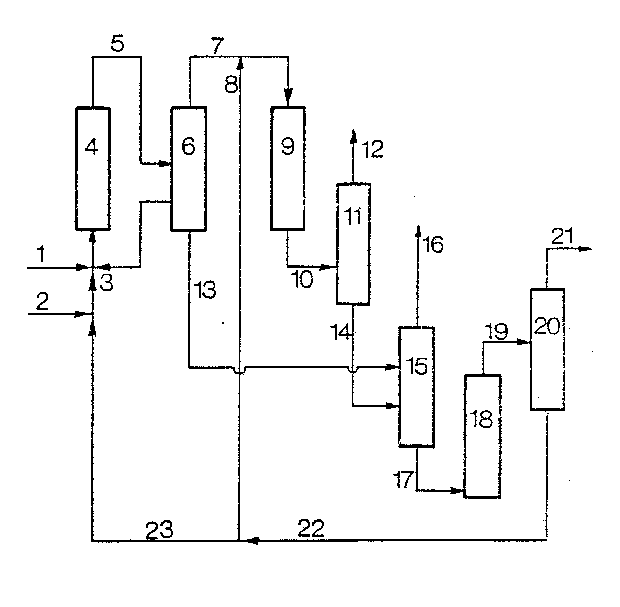

In der Figur wird eine beispielhafte Ausführungsform des erfindungsgemäßen Verfahrens schematisch erläutert. Das Isobuten enthaltende $C_4$-Kohlenwasserstoffgemisch (durch Leitung 1) und Methanol und ggf. der primäre $C_3$- oder $C_4$-Alkohol, z.B. Isobutanol (durch Leitung 2), werden vermischt, und die erhaltene Mischung wird durch Leitung 3 dem ersten Verätherungsreaktor 4 für die Verätherung zugeführt, in dem sich das saure Kondensationsmittel, z.B. ein Ionenaustauscher befindet. Zweckmäßig ist der Reaktor als Festbettreaktor, z.B. als Strömungsrohr oder Schlaufenreaktor oder Kombination der beiden Reaktortypen ausgestaltet. Es kann jedoch auch ein anderer Reaktor-Typ, z.B. ein Rührkessel oder eine Rührkesselkaskade, verwendet werden. Das erhaltene Reaktionsgemische wird aus dem Reaktor über Leitung 5 abgezogen und einer ersten Destillationskolonne 6 zugeführt. Am Kopf der Destillationkolonne wird ein isobutenarmes $C_4$-Kohlenwasserstoffgemisch-Raffinat durch Leitung 7 abgezogen. Gegebenenfalls wird über Leitung 18 aus Kolonne 6 eine Methanol enthaltende Fraktion über einen Seitenabzug abgezogen und in den Verätherungsreaktor 4 zurückgeführt. Das über Leitung 7 abgezogene, die nicht umgesetzten $C_4$-Kohlenwasserstoff enthaltende Raffinat der ersten Verätherungsstufe wird mit Isobutanol (aus Leitung 8) vermischt und einem zweiten Verätherungsreaktor 9 zugeführt, der wie der erste Verätherungsreaktor ausgestaltet sein kann. Das Reaktionsprodukt des zweiten Verätherungsreaktors 9 wird über Leitung 10 einer weiteren Destillationskolonne 11 zugeführt. Am Kopf dieser Destillationskolonne wird ein weitgehend Isobuten-, Alkohol- und Ätherfreies C₄-Kohlenwasserstoff-Raffinat über Leitung 12 abgezogen. Von den Sümpfen der Destillations-kolonnen 6 und 9 werden die die gebildeten tertiären Äther enthaltenden Sumpfprodukte über Leitungen 13 und 14 abgezogen und in die Destillationskolonne 15 geleitet. Es kann zweckmäßig sein, die beiden Ströme 13 und 14 an unterschiedlichen Stellen der Destillationskolonne 15 zuzuführen. Am Kopf der Kolonne 15 wird der Methyl-tert.-butyläther über Leitung 16 und das den C₃ oder C₄-Alkyl-tert.-butyläther und die gegebenenfalls im Übnerschuß zugesetzten C₃- oder C₄-Alkohol enthaltende Fraktion am Sumpf über Leitung 17 abgezogen und dampfförmig in den Zerlegungsreaktor 18 geleitet. Die Produkte aus dem Zerlegungsreaktor 18 werden über Leitung 19 in die Destillationskolonne 20 geleitet. Am Kopf von Kolonne 20 wird über Leitung 21 reines Isobuten abgezogen. Am Sumpf wird das bei der Zerlegung erhaltene Isobutanol über Leitung 22 abgezogen und über Leitungen 8 und 23 zur Verätherung zurückgeführt.

Beispiel

Die Verätherung wurde unter Verwendung eines C₄-Kohlenwasserstoffgemisches durchgeführt, welches den Rest (Raffinat) einer aus einer Äthylenanlage erhaltenen C₄-Fraktion darstellte, aus der das Butadien extrahiert worden war. Die Zusammensetzung des C₄-Kohlenwasserstoffgemisches nach der Butadien-Extraktion war wie folgt:

| | |
|---|---|
| Isobutan | 1,9 Vol.-% |
| n-Butan | 8,1 Vol.-% |
| Isobuten | 46,0 Vol.-% |
| Buten-1 | 26,7 Vol.-% |
| trans-Buten-2 | 10,1 Vol.-% |
| cis-Buten-2 | 7,0 Vol.-% |
| Butadien-1,3 | 0,2 Vol.-% |

Das C₄-Kohlenwasserstoffgemisch wurde mit der stöchiometrischen Menge (bezogen auf das enthaltene Isobuten) einer Mischung aus Methanol und Isobutanol im Molverhältnis 1:1 einem ersten Verätherungsreaktor zugeführt, der als Rohrreaktor ausgestaltet war. Dem Verätherungsreaktor wurde ein Temperaturprofil aufgeprägt, so daß die Austrittstemperatur des Reaktionsgemisches aus dem Verätherungsreaktor bei 40°C lag, während die Reaktionstemperatur im Reaktor in der Nähe der Zuführung der Ausgangsstoffe 70 bis 80°C betrug. Der Druck betrug 15 bar, so daß die Reaktion in flüssiger Phase ablief. Der Rohrreaktor war vollständig mit einem Ionenaustauscher in der Wasserstoform (sulfoniertes Polystyroldivinylbenzol-Harz, Lewatit SPC 118) gefüllt. Das Reaktionsgemische wurde in einer Destillationskolonne mit 15 praktischen Böden (Zulauf am 10. praktischen Boden) aufgetrennt Am Kopf der Kolonne wurde ein Methanol enthaltendes C₄-Kohlenwasserstoffgemische mit den nicht umgesetzten Kohlenwasserstoffen abgezogen, welches jeweils weniger als 100 Gew.-ppm Methyl-tert.-butyläther, Isobutanol und Isobutyl-tert.-butyläther enthielt. Am Sumpf wurde ein Gemisch aus Methyl-tert.-butyläther, Isobutanol und Isobutyl-tert.-butyläther abgezogen. Vom 5. praktischen Boden wurde eine methanolische Fraktion entnommen und zur ersten Verätherungsstufe zurückgeführt.

Das am Kopf der Destillationskolonne abgezogene C₄-Kohlenwasserstoffgemisch wurde mit der stöchiometrischen Menge Isobutanol vermischt und einem zweiten Verätherungsreaktor zugesetzt. Temperaturprofil und Druck wurden wie im ersten Verätherungsreaktor gewählt. Das Reaktionsprodukt wurde aus dem zweiten Verätherungsreaktor in einer zweiten Destillationskolonne mit 15 praktischen Böden mit Zulauf am 10. praktischen Boden destilliert. Am Kopf der zweiten Destillationskolonne wurde ein C₄-Kohlenwasserstoff-Raffinat entnommen, das jeweils weniger als 10 ppm Isobutanol, Isobutyltert.-butyläther und Methyl-tert.-butyläther enthielt. Der Methanolgehalt lag bei 0,5 Gew.-%. Das Sumpfprodukt der zweiten Destillationskolonne wurde mit dem Sumpfprodukt der ersten Destillationskolonne vereinigt. Die vereinigten Sumpfprodukte enthielten weniger als 100 ppm C₄-Kohlenwasserstoffe und wurden destillativ in ein Methyl-tert.-butyläther-Produkt und eine Fraktion aus Isobutanol und Isobutyl-tert.-butyläther aufgetrennt. Diese Fraktion wurde anschließend verdampft und in einen rohrförmigen Spaltreaktor geleitet. Die Zerlegungstemperatur wurde über den gesamten Reaktor zwischen 180 und 200°C gehalten, wobei die Austrittstemperatur bei 200°C lag. Als saurer Katalysator wurde mit Phosphorsäure getränktes und anschließend getempertes Kieselgel verwendet. Die Verweilzeit lag bei 3 s. Der Druck wurde auf 5 bar eingestellt.

Das Reaktionsprodukt aus dem Spaltreaktor wurde in einer weiteren Destillationskolonne mit 15 praktischen Böden mit Zulauf am 10. praktischen Boden aufgetrennt. Am Kopf der Kolonne wurde Isobuten mit einer Reinheit von mehr als 99,8% abgezogen. Die Nebenkomponenten waren n-Butene. Isobutanol oder Isobutyl-tert.-butyläther konnten jeweils bis unter 10 ppm abgetrennt werden. Methanol und Methyl-tert.-butyläther waren schon vor Einsatz in den Spaltreaktor destillativ abgetrennt worden, so daß sich eine Wasserwäsche erübrigte. Das Isobuten konnte ohne weitere Reinigung für weitere Reaktionen eingesetzt werden. Am Sumpf der Destillationskolonne wurde Isobutanol zurückgewonnen und zu den Verätherungsstufen zurückgeführt. Die Ausbeute an Isobuten und Methyl-tert.-butyläther lag bei über 97,5%, bezogen auf die Menge an Isobuten im eingesetzten C₄-Kohlenwasserstoffgemisch.

**Patentansprüche**

1. Verfahren zur gemeinsamen Herstellung von Methyl-tert.-butyläther und Gewinnung von Iso-buten aus Isobuten enthaltenden $C_4$-Kohlen-wasserstoffgemischen, dadurch gekennzeichnet, daß man

a) das $C_4$-Kohlenwasserstoffgemisch in Gegen-wart eines sauren Kohdensationsmittels in einer oder mehreren Verätherungsstufen mit Methanol und einem primären $C_3$- oder $C_4$-Alkohol umsetzt,

b) das erhaltene Reaktionsgemisch bzw. die erhaltenen Reaktionsgemische anschließend durch Destillation in
— eine die nicht umgesetzen Kohlenwasserstoffe enthaltende Fraktion,
— den Methyl-tert.-butyläther und
— eine den $C_3$- oder $C_4$-Alkyl-tert.-butyläther enthaltende Fraktion auftrennt und

c) dann den $C_3$- oder $C_4$-Alkyl-tert.-butyläther in Gegenwart eines sauren Katalysators bei erhöhten Temperaturen in Isobuten und primären $C_3$- oder $C_4$-Alkohol zerlegt.

2. Verfahren nach Anspruch 1, dadurch gekennzeichnet, daß man als saures Kondensationsmittel für die Ätherbildung Ionenaustauscher in der Wasserstofform verwendet.

3. Verfahren nach Anspruch 1 und 2, dadurch gekennzeichnet, daß das $C_4$-Kohlenwasserstoff-gemisch in mehreren vorzugsweise zwei, hintereinandergeschalteten Verätherungsstufen umgesetzt wird.

4. Verfahren nach Anspruch 3, dadurch gekennzeichnet, daß in der ersten Verätherungsstufe Methanol oder eine Mischung aus Methanol und dem primären $C_3$- oder $C_4$-Alkohol und in der zweiten Verätherungsstuge der primäre $C_3$- oder $C_4$-Alkohol eingesetzt werden.

5. Verfahren nach Anspruch 1 und 2, dadurch gekennzeichnet, daß das $C_4$-Kohlenwasserstoff-gemisch mit einer Mischung aus Methanol und dem primären $C_3$- oder $C_4$-Alkohol umgesetzt wird.

6. Verfahren nach Anspruch 3, daduch gekenn-zeichnet, daß das $C_4$-Kohlenwasserstoffgemisch in zwei hintereinandergeschlateten Verätherungsstufen mit jeweils einer Mischung aus Methanol und dem primären $C_3$- oder $C_4$-Alkohol umgesetzt wird, wobei das Verhältnis der der ersten Verätherungsstufe zugeführten Methanolmenge zu der ersten Verätherungsstufe zugeführten Menge an primärem $C_3$- oder $C_4$-Alkohol größer ist als das Verhältnis der der zweiten Verätherungsstufe zugeführten Methanolmenge zur der zweiten Verätherungsstufe zugeführten Menge an primärem $C_3$- oder $C_4$-Alkohol.

7. Verfahren nach Anspruch 1 bis 6, dadurch gekennzeichnet, daß die Verätherungsreaktion in ein oder zwei Verätherungsstufen erfolgt und bei zweistufiger Arbeitsweise bereits in der ersten Verätherungsstufe mit Methanol umgesetzt wird, wobei das Reaktionsgemisch der Verätherungsstufe bzw. bei zweistufiger Arbeitsweise das Reaktionsgemisch der ersten Verätherungsstufe einer Destillationszone mit Seitenabzug zugeführt wird, in der am Kopf eine die nicht umgesetzen $C_4$-Kohlenwasserstoffe enthaltende Fraktion am Sumpf eine den gebildeten tertiären Äther enthaltende Fraktion und am Seitenabzug eine nicht umgesetztes Methanol enthaltende Fraktion abgezogen werden und die das nicht umgesetzte Methanol enthaltende Fraktion in die Verätherungsstufe bzw. erste Verätherungsstufe zurückgeführt wird.

8. Verfahren nach Anspruch 1 bis 7, dadurch gekennzeichnet, daß das Reaktionsgemisch der zweiten Verätherungsstufe einer Destillation unterworfen wird, in der als Kopfprodukt eine die nicht umgesetzten $C_4$-Kohlenwasserstoffe enthaltende Fraktion mit einem Gehalt von jeweils höchstens 1000 Gew.-ppm $C_3$- oder $C_4$-Alkohol, Methyl-tert.-butyläther und $C_3$- oder $C_4$-Alkyl-tert.-butyläther und höchstens 1 Gew.-% Methanol abgezogen wird.

9. Verfahren nach Anspruch 1 bis 8, dadurch gekennzeichnet, daß das bei der Zerlegung des gegebenenfalls im Überschuß zugesetzten primä-ren $C_3$- oder $C_4$-Alkohol enthaltenden $C_3$- oder $C_4$-Alkyl-tert.-butyläthers erhaltene, Isobuten und den primären $C_3$- oder $C_4$-Alkohol enthaltende Reaktionsgemische einer Destillationszone zugeführt wird, in der als Kopfprodukt isobuten ohne Einschaltung einer Wasserwäsche mit einem Ge-halt an primärem $C_3$ oder $C_4$-Alkohol von höch-stens 500 Gew.-ppm abgezogen wird, und das erhaltene, den primären $C_3$- oder $C_4$-Alkohol enthaltende Bodenprodukt in die Verätherung zurückgeführt wird.

**Revendications**

1. Procédé pour la préparation d'éther méthyl-tertiobutylique et le récupération simultanée d'isobutène à partir de mélanges d'hydrocarbures en $C_4$ contenant de l'isobutène, caractérisé en ce que:

a) le mélange d'hydrocarbures en $C_4$ est mist en réaction avec du méthanol et un alcool primaire en $C_3$ ou $C_4$, en présence d'un agent de condensation acide, dans un ou plusieurs étages d'éthérification,

b) le mélange réactionnel ou les mélanges réactionnels obtenus sont ensuite séparés par distillation en
— une fraction contenant les hydrocarbures n'ayant pas réagi,
— l'éther méthyl-tertiobutylique et
— une fraction contenant l'éther alcoyl(en $C_3$ ou $C_4$)-tertiobutylique, et

c) l'éther alcoyle(en $C_3$ ou $C_4$)-tertiobutylique est ensuite décomposé en isobutène et en alcool primaire en $C_3$ ou $C_4$ en présence d'un catalyseur acide et à température élevée.

2. Procédé selon la revendication 1, caractérisé en ce qu'on utilise, en tant qu'agent de condensation acide pour la formation de l'éther, des échangeurs d'ions sous la forme hydrogénée.

3. Procédé selon la revendication 1 ou 2, caractérisé en ce que le mélange d'hydrocarbures en $C_4$ est mis en réaction dans plusieurs, de préférence deux étages d'ethérification disposés en série.

4. Procédé selon la revendication 3, caractérisé en ce qu'il est introduit, dans le premier étage d'éthérification, du méthanol ou un mélange de méthanol et de l'alcool primaire en $C_3$ ou $C_4$ et, dans le second étage d'éthérification, l'alcool primaire en $C_3$ ou $C_4$.

5. Procédé selon la revendication 1 ou 2, caractérisé en ce que le mélange d'hydrocarbures en $C_4$ est mis en réaction avec un mélange de méthanol et de l'alcool primaire en $C_3$ ou $C_4$.

6. Procédé selon la revendication 3, caractérisé en ce que le mélange d'hydrocarbures en $C_4$ est mis en réaction, dans deux étages d'éthérification diposés en série, avec chaque fois un mélange de méthanol et de l'alcool primaire en $C_3$ ou $C_4$, le rapport de la quantité de méthanol introduite dans le premier étage d'éthérification à la quantité d'alcool primaire en $C_3$ ou $C_4$ introduite dans le premier étage d'éthérification étant plus grand que le rapport de la quantité de méthanol introduite dans le second étage d'éthérification à la quantité d'alcool primaire en $C_3$ ou $C_4$ introduite dans le second étage d'éthérification.

7. Procédé selon l'une quelconque des revendications 1 à 6, caractérisé en ce que la réaction d'éthérification est effectuée dans un ou deux étages d'éthérification et, en cas de mode opératoire en deux étages, le mélange d'hydrocarbures est mis en réaction avec le méthanol dès le premier étage d'éthérification, le mélange réactionnel de l'étage d'éthérification ou, en cas de mode opératoire en deux étages, le mélange réactionnel du premier étage d'éthérification étant dirigé vers une zone de distillation à soutirage latéral, dans laquelle il est extrait, en tête, une fraction qui contient les hydrocarbures en $C_4$ n'ayant pas réagi, au fond, une fraction qui contient l'éther tertiaire formé et par le soutirage latéral, une fraction qui contient le méthanol qui n'a pas réagi, la fraction qui contient le méthanol n'ayant pas réagi étant renvoyée dans l'étage d'éthérification ou dans le premier étage d'éthérification.

8. Procédé selon l'une quelconque des revendications 1 à 7, caractérisé en ce que le mélange réactionnel du second étage d'éthérification est soumis à une distillation, ou cours de laquelle il est extrait, en tant que produit de tête, une fraction contenant les hydrocarbures en $C_4$ n'ayant pas réagir, avec une teneur de 1000 ppm en poids au maximum de chacune des substances alcool en $C_3$ ou $C_4$, éther méthyl-tertiobutylique et éther alcoyle(en $C_3$ ou $C_4$)-tertiobutylique et de 1% en poids au maximum de méthanol.

9. Procédé selon l'une quelconque des revendications 1 à 8, caractérisé en ce que le mélange réactionnel qui contient de l'isobutène et l'alcool primaire en $C_3$ ou $C_4$ et qui provient de la décomposition de l'éther alcoyl(en $C_3$ ou $C_4$)-tertiobutylique contenant éventuellement de l'alcool primaire en $C_3$ ou $C_4$ introduit en excès, est dirigé vers une zone de distillation dans laquelle il est extrait, en tant que produit de tête, de l'isobutène sans intercalation d'un lavage à l'eau, ayant une teneur en alcool primaire en $C_3$ ou $C_4$ de 500 ppm en poids au maximum, le produit de fond obtenu, qui contient l'alcool primaire en $C_3$ ou $C_4$, étant renvoyé à l'éthérification.

**Claims**

1. A process for conjointly preparing methyl tert.-butyl ether and obtaining isobutene from an isobutene-containing $C_4$-hydrocarbon mixture, wherein

a) the $C_4$-hydrocarbon mixture is reacted, in one or more etherification stages, with methanol and a primary $C_3$- or $C_4$-alcohol in the presence of an acidic condensing agent,

b) the resulting reaction mixture or the resulting reaction mixtures are then separated by distillation into a fraction containing the unconverted hydrocarbons, the methyl tert.-butyl ether, and a fraction containing the $C_3$- or $C_4$-alkyl tert.-butyl ether, and

c) thereafter the $C_3$- or $C_4$-alkyl tert.-butyl ether is decomposed at an elevated temperature, in the presence of an acidic catalyst, into isobutene and primary $C_3$- or $C_4$-alcohol.

2. A process as claimed in claim 1, wherein an ion exchanger in the hydrogen form is used as the acidic condensing agent for the formation of the ether.

3. A process as claimed in claims 1 and 2, wherein the $C_4$- hydrocarbon mixture is reacted in several, preferably two, successive etherification stages.

4. A process as claimed in claim 3, wherein methanol, or a mixture of methanol and the primary $C_3$- or $C_4$-alcohol, is employed in the first etherification stage, and the primary $C_3$ or $C_4$-alcohol is employed in the second etherification stage.

5. A process as claimed in claims 1 and 2, wherein the $C_4$-hydrocarbon mixture is reacted with a mixture of methanol and primary $C_3$- or $C_4$-alcohol.

6. A process as claimed in claim 3, wherein the $C_4$-hydrocarbon mixture is reacted with a mixture of methanol and the primary $C_3$- or $C_4$-alcohol in each of two successive etherification stages, the ratio of the amount of methanol fed to the first etherification stage to the amount of primary $C_3$- or $C_4$-alcohol fed to the first etherification stage being greater than the corresponding ratio of the materials fed to the second etherification stage.

7. A process as claimed in claims 1 to 6, wherein the etherification reaction is carried out in one or two stages, and in the case of two-stage operation the reaction with methanol is carried out in the first stage, and the reaction mixture from the etherification stage or, in two-stage operation, the reaction mixture from the first etherification stage is fed to a distillation zone with side take-off, in which zone a fraction containing the unconverted $C_4$-hydrocarbons is taken off overhead, a fraction containing the tertiary ether formed is taken off at the bottom and a fraction containing the unconverted methanol is taken off at the side, the fraction containing the unconverted methanol being recycled to the etherification stage or to the first etherification stage.

8. A process as claimed in claims 1 to 7, wherein the reaction mixture from the second etherification stage is subjected to a distillation, in which the overhead product taken off is a fraction which contains the unconverted $C_4$-hydrocarbons, together with not more than 1% by weight of methanol and not more than 1,000 ppm by weight of each of the following: $C_3$- or $C_4$-alcohol, methyl tert.-butyl ether and $C_3$- or $C_4$-alkyl tert.-butyl ether.

9. A process as claimed in claims 1 to 8, wherein the reaction mixture which is obtained on decomposition of the $C_3$- or $C_4$-alkyl tert.-butyl ether (containing any excess added primary $C_3$- or $C_4$-alcohol), and which contains isobutene and the primary $C_3$- or $C_4$-alcohol is fed to a distillation zone in which — without interpolating a water wash — isobutene containing not more than 500 ppm by weight of primary $C_3$ or $C_4$-alcohol is taken off over-head, whilst the bottom product obtained, which contains the primary $C_3$ or $C_4$-alcohol, is recycled to the etherification.